# EUROPEAN PATENT APPLICATION

(11) **EP 1 785 267 A1**
(43) Date of publication of application: **16.05.2007**
(21) Application number: 05256968.8
(22) Date of filing: 11.11.2005
(51) Int. Cl.: B32B 25/10, B32B 37/15, B32B 37/14

(54) **Elastic laminate**

(71) Applicant: Innovative Elastics Ltd., Limerick (IE)
(72) Inventor: Marchal, Daniel, 9142 Burden (LU); Burke, David, Limerick (IE); Flynn, Edward, Limerick (IE)
(74) Representative: Jones, Helen M.M.

(57) **Abstract**

A method for producing an elastic laminate which behaves anisotropically upon stretching comprising the steps of: a. contacting a non-woven fabric web (5) which is elastically extendable in one direction only with a vulcanizable rubber sheet (3) to form a composite laminate; b. feeding the composite laminate between nip rollers (6); and c. vulcanizing the rubber of the composite laminate to produce an elastic laminate which is elastically stretchable by at least 100% in one direction only.

## Description

The present invention relates to elastic laminates which are stretchable in one direction only and are useful in the fields of disposable hygiene and medical products.

The use of elastic laminates is widespread, particularly in the fields of disposable hygiene and medical products. By laminating non-woven materials to a central core of elastomer film, it is possible to obtain an appropriate balance of elasticity whilst retaining the strength and feel of the non-woven material. Such laminates are often used in absorbent articles such as diapers. In many applications, an elastic film, tape or thread is combined with a non-woven, non-stretchable fabric. In one type of product, the elastic component is stretched prior to attachment to the unstretched non-woven fabric such that when the elastic material is relaxed, the non-woven material is gathered. An example of such a laminate can be seen in the elastication in the leg (leg gather) of baby diapers.

An example of a material which may be employed as an elastic laminate is vulcanized rubber. Conventionally where vulcanized rubber is used as the elastic laminate, it would be the usual practice that the rubber is vulcanized prior to being contacted with the non-woven layers. The rubber layers would be wound onto a drum and then, following vulcanization, cut into strips which are then bonded to non-woven fabrics by means of hot melt adhesive or ultrasonic lamination.

An alternative method employed to impart an elasticity to the resulting laminate is to adhere a non-prestretched elastic component to a non-stretchable non-woven fabric and to break the fibres of the non-woven fabric to allow the laminate to stretch. For example, in EP-B-783405, a method wherein slits are generated in the non-woven layer is described.

It is now possible to obtain commercially non-woven fabrics which are elastically stretchable in one direction only. An example of such non wovens are those detailed in US 5486411. Following the advent of such non-woven fabrics, research has focussed on producing laminates which use such materials. These non-woven materials have the advantage that they have stretch properties without having to compromise the structures of the material. In WO-A-02/18125, for example, a method wherein a laminate which is elastic in the machine direction but not in the cross-direction is produced is described. A non-woven which is elastically extendable by at least 25% in the cross direction is laminated to a plurality of elastic members at spaced apart locations.

As used hereinafter, the term "machine direction" refers to the direction in which the material moves through production apparatus and the term "cross direction" defines the direction in the plane of the material which is perpendicular to the machine direction.

Where a non-woven is attached to an elastomer layer, it is usual that the adhesion is either by use of a suitable adhesive or by the use of thermal lamination wherein adhesion is achieved by slight melting of the elastomer layer. Where an adhesive is required, this may be undesirable due to the difficulty in selecting an appropriate adhesive and the associated costs. Furthermore, where an adhesive is required, this adds an additional step to the process and impacts negatively on elasticity and costs. An example of thermal lamination is ultrasonic lamination. Ultrasonic lamination is also an expensive technique and the result will be a laminate which is bonded at discrete rupture points which may not be appropriate for all products. Such weak points may limit the stretchability of the elastic.

While there have been various developments in the production of elastic laminates, it is apparent that there is still a need for a simple and effective method by which an elastic laminate which is stretchable in one direction only can be produced. Ideally such a method would avoid the use of expensive adhesive and ultrasonic lamination techniques.

The present invention provides a method for producing an elastic laminate which behaves anisotropically upon stretching comprising the steps of:
(a) contacting a non-woven fabric web which is elastically extendable in one direction only with a vulcanizable rubber sheet, to form a composite laminate;
(b) feeding the composite laminate between nip rollers;
(c) vulcanizing the unvulcanized rubber of the composite laminate to produce an elastic laminate which is elastically stretchable by at least 100% in one direction only.

The term elastically extendable" as used herein refers to the non-woven fabric web and is used to describe the situation wherein after stretching the material returns to its original shape and maintains its structural integrity such that there is no breakage or rupturing of fibres. The term "elastically stretchable" is used to describe the elastic laminate produced and defines the situation wherein after stretching the material returns to its original shape because of the low permanent set of the vulcanised elastic and maintains its structural integrity such that there is no breakage or rupturing of material.

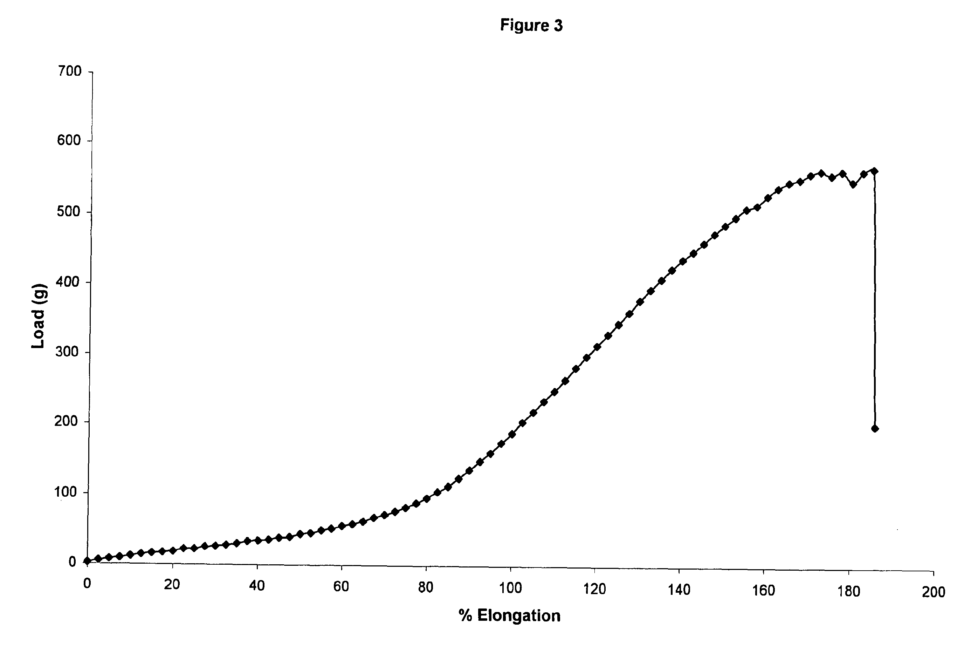

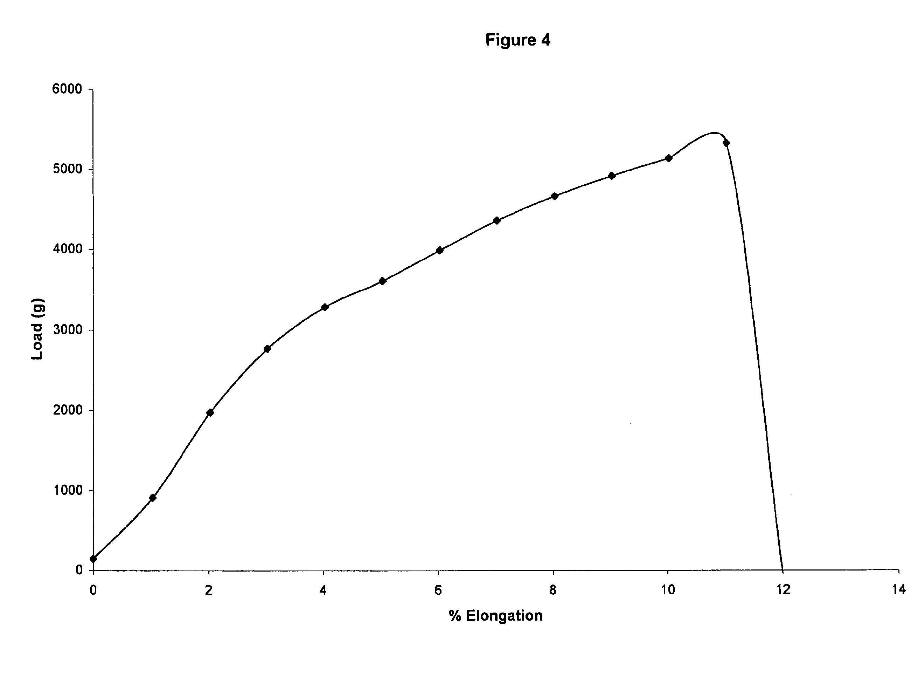

As detailed above the non-woven fabric web is elastically extendable in one direction only and the elastic laminate product is elastically stretchable in one direction only. These properties are measured by, for example, ASTM method D882-02. With regard to the non-woven fabric web, when an attempt is made to stretch it in a direction which is not the one in which it is elastically extendable there will be minimal movement until the yield point of the material is reached, at which point tearing and rupturing of the fibres occurs the behaviour of the non-woven fabric web when stretched in both the machine and cross-direction is illustrated schematically in Figures 3 and 4 respectively. For generating this data, the non-woven fabric web was SHAPE 30 available from Fibertex, Denmark. This non-woven fabric web has a basis weight of 30 gm⁻² and is based on modified polypropylene. The sample tested had a width of 2.54cm (1 inch) and was elastically extendable in the cross direction. It can be seen that the non-woven fabric web tears at a significantly lower elongation when stretched in the direction in which it is not elastically extendable (i,e, the machine direction).

The tensile strength of the non-woven fabric web depends in part on the basis weight. As an indication, where non-woven fabric web with a basis weight of 15gm⁻² is used, preferably the tensile strength in the direction in which the fabric web is elastically extendable is in the range from 4 to 7N/50mm. Preferably the tensile strength in the direction in which the fabric web is not elastically extendable is in the range from 60 to 100N/50mm. These values are as measured according to test method ERT 20.2-89. The elongation at break in the elastically extendable direction is preferably in the range from 160-260% and in the non-elastically extendable direction, it is in the range from 15 to 25%.

Where the non-woven fabric web has a basis weight of 100gm⁻², preferably the tensile strength in the direction in which the fabric web is elastically extendable is in the range from 20 to 35N/50mm and the tensile strength in the direction in which the fabric web is not elastically extendable is in the range from 110 to 150N/50mm. The elongation at break in the elastically extendable direction is preferably in the range from 230 to 270% and in the non-elastically extendable direction it is in the range from 30 to 35%.

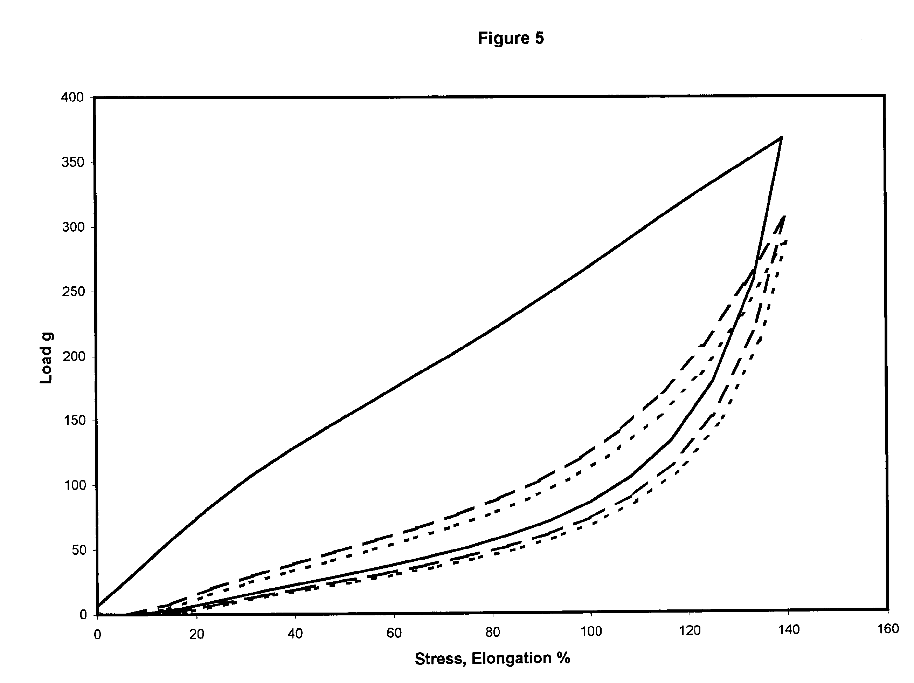

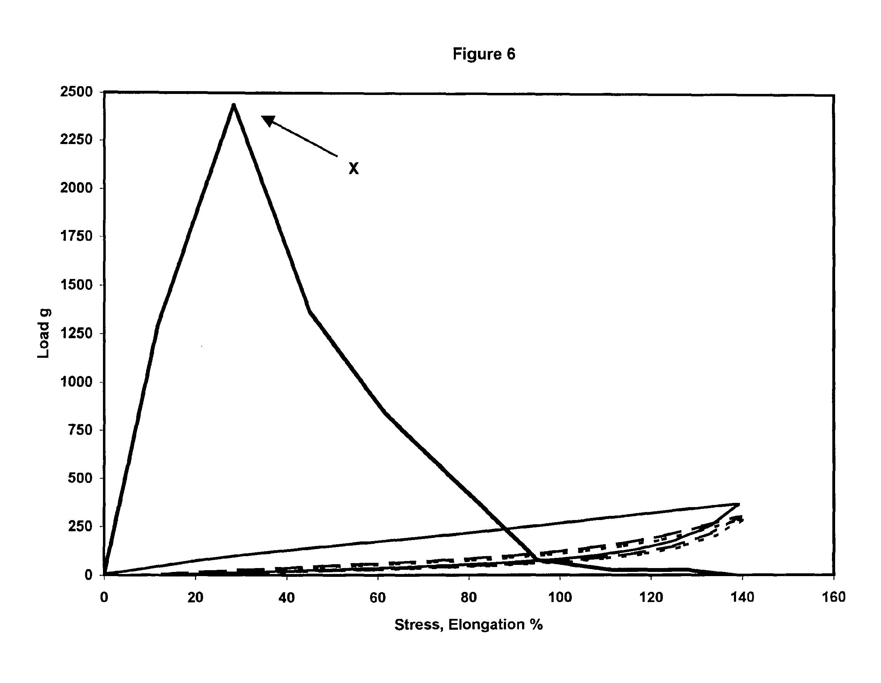

Similarly, when an attempt is made to stretch the elastic laminate in a direction which is not the one in which it is elastically stretchable, initially there is minimal movement. At the point where the force overcomes the adhesion between the non-woven fabric web and the rubber sheet, tearing of the non-woven fabric web occurs and it separates from the rubber sheet. This occurs at an elongation in the range from 20 to 40%. The results of tensile tests carried out on the elastic laminate are illustrated in Figures 5 and 6. The laminate tested was formed from shape 30 non-woven fabric web available from Fibertex, Denmark and the elastic material used was "System 7000" (TM) available from Fulflex. The sample tested had a width of 2.54cm (1 inch).

The term "anisotropic" is used to describe a behaviour which is different in different directions upon stretching.

In step (a) of the present method, a non-woven fabric web is contacted with a vulcanizable rubber sheet (film). Preferably the non-woven fabric stock has a basis weight in the range from 10 to 100gm⁻². As detailed above the non woven fabric web is elastically extendable in one direction only. Suitable materials for the non-woven fabric web include Shape PP Non Woven (TM) which are available from FiberTex A/S of Denmark and Elaxus (TM) which is available from Golden Phoenix of Taiwan. Preferably it is the cross direction in which the non-woven fabric web is elastically extendable. The only way such a material could be stretched in the machine direction is by breaking the fibres which does not fall within the definition of elastically stretchable according to the present invention. In particular, Shape PP Non Woven with a basis weight of 15 or 30gm⁻² is preferred for use as the non-woven fabric web.

As mentioned above, where vulcanized rubber is used as the elastic laminate, it is usual that it is vulcanized prior to being contacted with the non-woven layers. In contrast to this conventional method, the present Applicant has discovered that surprisingly it is possible to produce a high quality elastic laminate by vulcanizing the rubber layer after it has been contacted with the non-woven layer, thus avoiding the need for any adhesive.

The vulcanizable rubber sheet generally comprises a cross-linking agent in addition to a cross-linkable polymer. Examples of suitable cross-linking agents include sulfur either alone or in combination with thiocarbonmates and accelerators such as mercaptobenzothiazole and sulfenamides. Other suitable crosslinking agents include activators such as zinc oxide and stearic acid. Prior to vulcanization, the rubber will be soft and exhibit viscous liquid type properties. This means that it is tacky and upon contact with the non-woven fabric web there will be a degree of mechanical attachment by interpenetration between the rubber sheet (film) and the non-woven fabric web. The vulcanizable rubber sheet has upper and lower faces for contacting with the non-woven fabric web.

A web of the non-woven fabric may be brought into contact with just the upper or lower face of the vulcanizable rubber sheet. Alternatively a web of non-woven fabric may be contacted with both the lower and upper faces of the vulcanizable rubber stock such that the resulting composite comprises a central vulcanizable rubber layer sandwiched between two layers of non-woven fabric sheet. Thus in one embodiment of the present invention, in step (a), two non-woven fabric webs are contacted with the vulcanizable rubber sheet, one with the upper face and one with the lower face.

Preferably the vulcanizable rubber sheet has a thickness in the range from 0.05 to 0.2mm.

Examples of suitable sources of vulcanizable rubber for use as the vulcanizable rubber sheet include both natural rubbers and synthetic rubbers. The vulcanizable rubber compounds for was in the present invention may be formed by compounding raw polymeric rubber materials with various addition.

An example of a preferred vulcanizable rubber for use as the vulcanizable rubber sheet is a blend of polyisoprene (either natural or synthetic) and other ingredients including titanium dioxide and a cross-linking agent. Suitable examples of natural rubber for use as the crosslinkable polymer in the vulcanizable rubber sheet include Standard Malaysian Rubber such as SMR5L or similar grades from Indonesia, Sri Lanka, Ivory Cost, Cameroon, Vietnam, Nigeria and Cambodia. These rubber sources are the raw rubber material which do not include a cross-linked agent. In these cases it is therefore necessary to separately add a cross-linking agent.

Examples of suitable synthetic rubbers for use as the crosslinkable polymer in the vulcanizable rubber sheet include polyisoprene and styrene butadiene. Commercially available examples of polyisoprene are Nippol 2000 from Nippon Zeon, Ski-3 and Ski-35 (TM) from Togliatti, Russia and Kraton Ir-305, Ir-309 and Ir-310 (TM) from Kraton Polymers, Holland. Where a styrene butadiene is used as the cross-linkable polymer in the vulcanizable rubber sheet, examples of suitable materials are Tufdene (TM) from Asahi Kasei (Japan), SBR IS02 (TM) from Dow, Holland and Intol1502 (TM) available from Polimeri Europa UK. As noted above, these are raw rubber material sources and as such, do not include a cross-linking agent. It is therefore necessary, in these cases to separately add a cross-linking agent.

In an embodiment of the present invention where the non-woven fabric is only contacted with either the upper or lower face of the vulcanizable rubber sheet, the method may include a further step of coating the face which is not contacted with non-woven fabric layer with an anti-blocking material so as to prevent any adhesion. Examples of suitable anti-blocking agents include talc, starch or mixtures of the two. The intention of including an anti-blocking agent is to prevent sticking or adhesion.

The rubber material used to form the vulcanizable rubber sheet may be obtained in the form of sheets or in some cases it may be in the form of unprocessed slabs or chunks. Depending on the form of the vulcanizable rubber material, it first needs to be calendered such that it is of an appropriate thickness for further processing. This can be achieved by passing the rubber between calendering rolls which are heated to a temperature in the range from 100 to 150°C and move at a speed of up tp 50mm/min. The calendered rubber is then passed round cooling drums at a temperature in the range from 10 to 15°C. The rubber is then ready to be used in the method of the present invention.

Due to the viscous properties of the vulcanizable rubber, some of the fibres of the non-woven fabric web will penetrate into the rubber. This is known as mechanical entanglement or interpenetration. Rather than any chemical bonds being formed, the interaction between the two layers is by way of physical forces.

After contacting the two webs together, the resulting composite laminate is fed between nip rollers. Feeding the composite laminate between nip rollers causes the non-woven layer and the vulcanizable rubber sheet to adhere to one another. The Applicant has surprisingly found that the degree of adhesion achieved between the non-woven webs and the vulcanizable rubber is sufficiently strong to enable the composite to be suitable for the intended uses such as in the waist and leg panels of diapers where the composite will remain in contact when subjected to the movement of the wearer. In preference to the two layers separating, the composite will stretch elastically. The appropriate adhesive strength can be tested by a Peel Test on a 25mm sample of the laminate according to ASTM method D4393-04.

The non-woven is preferably adhered across the entire surface of the vulcanizable rubber stock rather than at just discrete points as compared to techniques involving welding, such as ultrasonic lamination. In a welding technique, at the points where heat is applied, the rubber layer becomes decomposed and the non-woven layer melted. This may result in a weakening of the final laminate. By ensuring that adhesion is achieved across the entire surface, this avoids the situation in, for example, a welding (e.g ultrasonic welding) technique where, at the welding points, the rubber layer is decomposed and the non-woven layer melted. In the event that a specific bonding pattern is, however, required, this can be achieved by using a patterned nip roller. Thus in the method of the present invention, adhesion is achieved without compromising the structural integrity of the layers in contact and without imparing the elasticity of the structure such as happens in adhesive joining or ultrasonic welding.

In step (c) of the method of the present invention, the vulcanizable rubber layer as now adhered to the non-woven fabric stock, is subjected to a vulcanization step. Vulcanization causes the rubber layer to crosslink but as the Applicant has surprisingly found that the conditions used for vulcanization does not affect the physical properties of the non-woven fabric web(s) specifically its elastic properties, softness and strength. Vulcanization may be carried out by a number of methods and the method of the present invention is not limited by reference to any particular vulcanization step. Irrespective of the method used, the rubber layer will no longer exhibit viscous liquid type properties or be tacky.

Vulcanization is carried out by subjecting the tacky rubber which comprises a mixture of cross-linkable elastomer and a vulcanizing agent, to conditions under which crosslinking takes place. After vulcanization, the rubber layer is no longer tacky. Preferably raised temperature or a combination of raised pressure and temperature such as in an autoclave is used. Where a raised temperature is used preferably the temperature is in the range from 100 to 170°C.

As an illustration, in one embodiment of the present invention, in the vulcanization step (c), layers of the composite laminate obtained in step (b) are wound on to a metal drum. The metal drum, will usually have a size in the range from 0.5 to 1.9m (20 to 72 inches), preferably approximately 1.3m (52 inches). The metal drum is then heated in an oven to a temperature in the range of 120 to 160°C.

In a further embodiment of the present invention, vulcanization is carried out by use of electron beam technology wherein an electron beam is directed at the vulcanizable rubber to achieve cross-linking. Electron beam technology is widely used in the paper and printing industries to cure (i.e. cross-link) coatings and lacquers. When the uncured elastomeric polymer is exposed to highly accelerated electrons, chemical bonds are formed between polymer molecules. Electron beams are available in a variety of widths and sizes capable of handling sheets, films or webs of 1.5 metres width or greater. As an example, a suitable electron beam dose for achieving curing in the present application is approximately 220kgray.

After vulcanization, an elastic laminate with anisotropic behaviour is obtained. The elastic laminate has an elastic stretchability of at least 100% in one direction only. In a preferred embodiment, the stretchability of the elastic laminate obtained is at least 150% more preferably, at least 200%. The anisotropic behaviour, more specifically the stretchability of the elastic laminate and the direction in which it is stretchable are determined by the nature of the non-woven fabric stock. Therefore in order to ultimately obtain an elastic laminate with the above detailed elastic stretchability, the non-woven fabric stock must also have this elastic extendability. When the elastic laminate is stretched in the direction which it does not exhibit and elastic stretchability, initially there is very little movement upon the application of a force. Once the yield point is reached however, the non-woven fabric web breaks and comes away from the vulcanized rubber sheet at the break point.

By the method of the present invention an elastic laminate with an anisotropic behaviour, i.e. stretchable in one direction only is obtained without the need for any separate adhesive layer or welding technique. The present application is also directed to this product in itself as obtained by the present method. In this regard, the present invention provides an elastic laminate which has an elastic stretchability of at least 100% in one direction only and which comprises:
a vulcanized rubber layer having upper and lower faces;
a non-woven fabric layer attached to either or both of the upper and lower faces of the vulcanized rubber layer wherein the non-woven fabric layer(s) is mechanically entangled with the vulcanized rubber layer.

Where only one side of the rubber film is adhered to the non-woven fabric, it is generally necessary to coat the other face with either a blocking layer of talc or alternatively a non-adhesive layer.

The elastic laminate of the present invention may be in the form of strips or alternative shapes for use in the end application in question. If required, the product may also include apertures. Where apertures are present, these may be created in the elastic laminate by use of laser, needle punching or ultrasonic treatment or slits in the cross or machine direction as seen in perforated paper.

The elastic laminate of the present invention is particularly appropriate for use in the production of disposable hygiene products. By achieving stretchability in one direction onlywhilst retaining the properties of a non-woven fabric, the elastic laminate detailed in particularly lends itself to use in the side sealing tapes, waistbands and side-panels of diapers which need to respond elastically to the movement of the wearer. The elastic laminate of the present invention is also useful in the production of disposable medical products including stretchable bandages and stretchable wound dressings. As one can envisage, the potential uses of such an elastic laminate are numerous.

### FIGURES

Figure 1 is a schematic diagram of the method of the present invention.
Figure 2 is a graphical representation, with the vertical dimension greatly exaggerated, of an elastic laminate of the present invention.
Figure 3 shows the results of tensile tests carried out according to ASTM D882-02 on a non-woven fabric web in the cross-direction;
Figure 4 shows the results of tensile tests carried out according to ASTM D882-02 on a non-woven fabric web in the machine direction;
Figure 5 illustrates the results of tensile tests carried out according to ASTM 0882-02 on an elastic laminate according to the present invention, in the cross direction;
Figure 6 illustrates the results of tensile tests carried out according to ASTM D882-02 and cross direction on an elastic laminate according to the present invention, in the machine direction and cross direction;
Figure 7 shows the results of peel tests carried out according to ASTM D4393-04 on an elastic laminate according to the present invention in the cross direction; and
Figure 8 shows the results of peel tests carried out according to ASTM D4393-04 on an elastic laminate according to the present invention in the machine direction.

The present invention will now be illustrated by reference to Examples. These examples are in no way intended to limit the scope of protection claimed.

### EXAMPLES

A vulcanizable rubber starting material (1) with the following composition:

| | |
|---|---|
| Polyisoprene rubber | 100 phr |
| Mineral fillers | 0-25phr |
| Sulphur | 0.5-2phr |
| Curing accelerators | 0.5-2phr |
| anti-oxidants | 0-2phr |
| Zinc oxide | 2-8phr |
| pigment | 0-8phr |

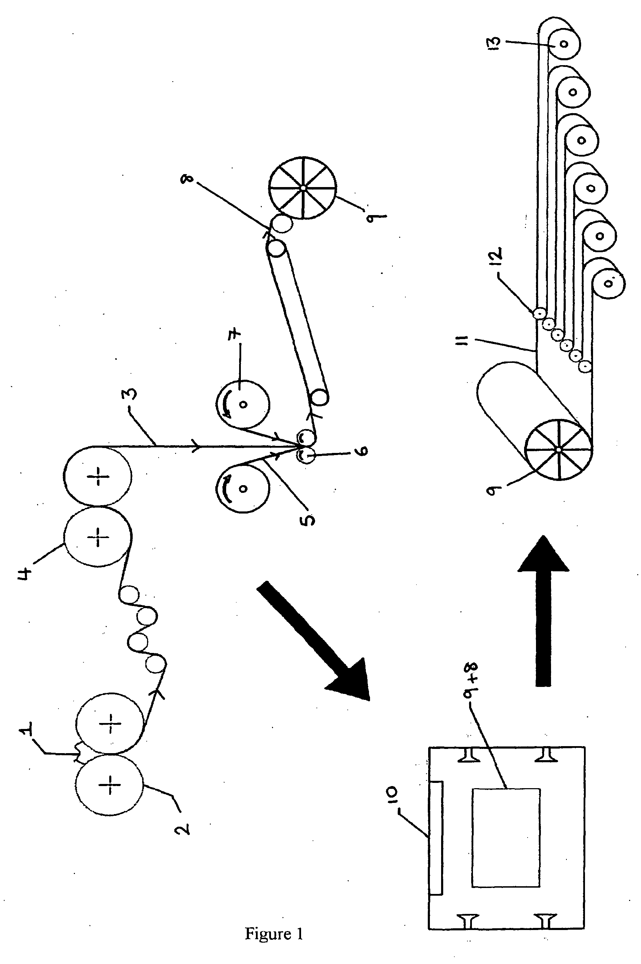

was passed between two calender rolls (2) as shown in Figure 1. The speed of the calender rolls was 16m/min and they were heated to a temperature of 130°C. The rubber sheet (3) leaving the calender rolls had a thickness of 0.1mm. The rubber sheet was then passed around cooling drums (4) moving at a speed of 18m/min and at a temperature of 12°C. The width of the rubber sheet (3) was 760mm.

The rubber sheet (3) and two feeds of non-woven fabric web (5) were then fed to nip rollers (6). The direction in which the rubber sheet is fed through the apparatus is defined as the machine direction. The direction in the plane of the rubber sheet which is perpendicular to the machine direction is defined as the cross-direction. The non-woven fabric web (5) used had a basis weight of 30gm⁻² and a width of 800mm. The feed rolls (7) held a total of 100 m minimum length of non-woven fabric web each.

The nip rollers (6) were covered with a rubber coating which was 8mm thick and a Shore hardness of 45. As measured according to ASTM D2240. The forward force exerted by the nip rollers was approximately 60kg. In this example, a total of 400m of laminate (8) was wound onto drum (9) at a speed of 20m/min.

Prior to vulcanization the drum of wound laminate was wrapped with three layers of polypropylene film and six layers of textile fabric. The layers of polypropylene and textile fabric are wound around the drum in order to prevent oxidation by air of the rubber during the process. The drum of laminate was then heated in a hot air oven (10) at a temperature of 143°C for 3.5 hours. After cooling to less than 100°C for a period of 2 to 3 hours, the vulcanized laminate (11) was unwound, slit using rotating knives (12) and subsequently wound into rolls (13) as shown in Figure 1.

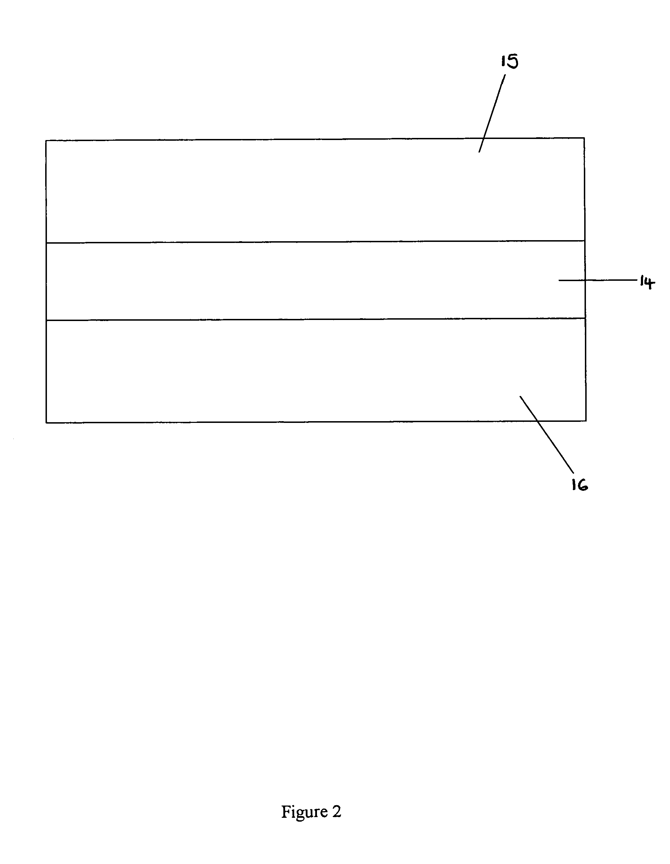

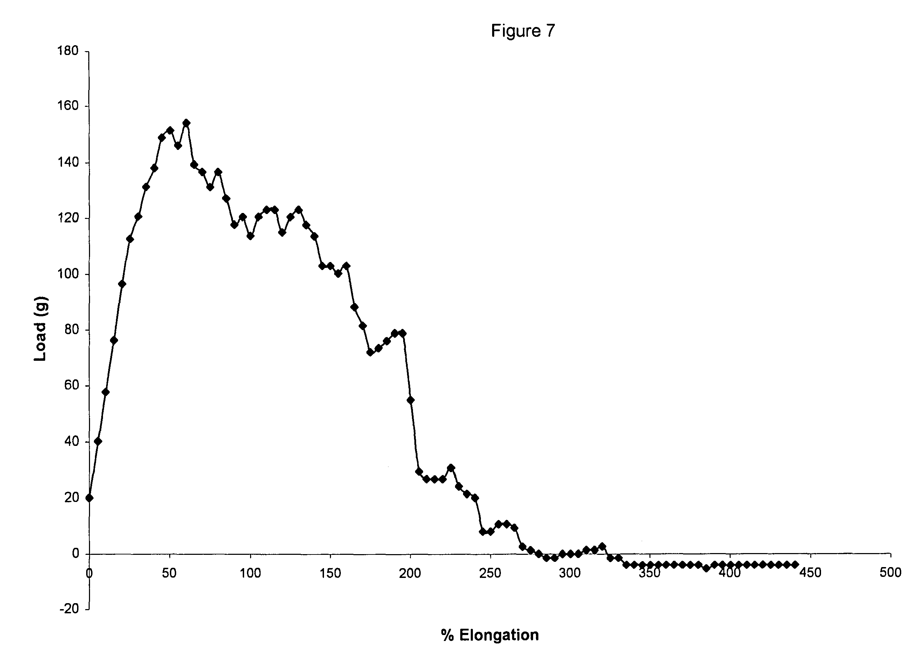

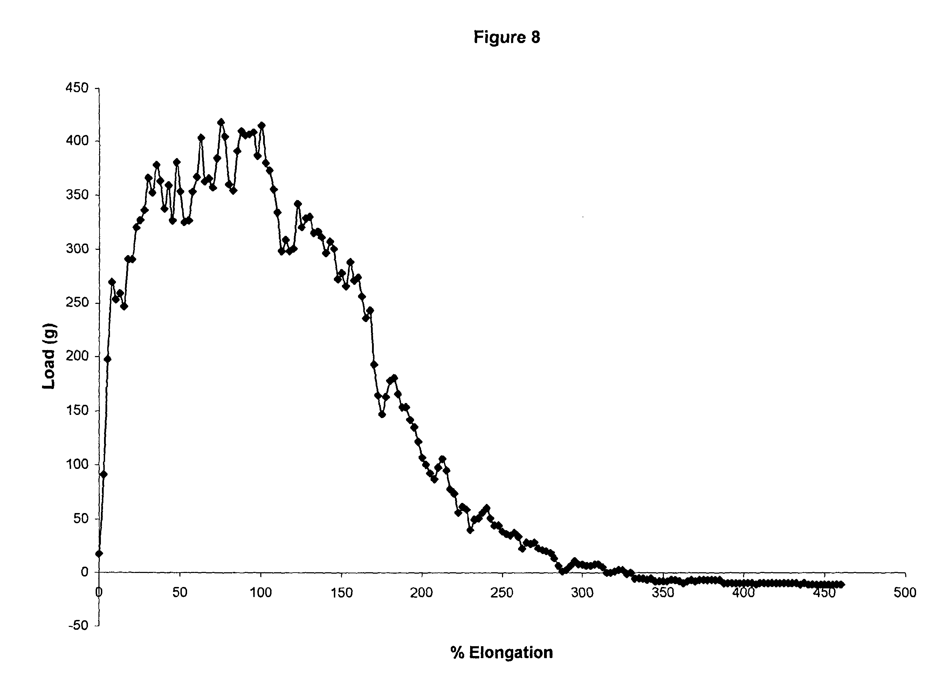

The laminate obtained is illustrated in Figure 2. It can be seen that it comprises a central vulcanised rubber layer (14) to which a non-woven fabric layer is adhered (15,16) on both the upper and lower faces. The laminate was subjected to a peel test. In this test, a 25mm sample of the laminate was tested according to ASTM D4393-04 on a laboratory scale. The results of the peel tests are shown in Figures 7 and 8. The peak of the graphs corresponds to the delamination strength and elongation. In the cross-direction the non-woven fabric layer tore at a force of 340g and extension of 180%. In the machine direction, the non-woven fabric layer tore at a force of 1020 and extension of 30%. Tearing occurs in preference to peeling because the adhesive force between the two layers is stronger than the non-woven fabric web.

Tensile tests were also carried out on a sample with a width of 6.35mm. The laminate was prepared according to the method detailed above with the exception that the non-woven fabric web had a basis weight of 25gsm. The rubber sheet (in this case 2L39 film available from Fulflex International Company had a thickness in the range from 70 to 90µm. The laminate was tested in both the machine and cross directions. The results are illustrated in Figures 5 and 6. In the cross direction, the sample was stretched three times.

## Claims

1. A method for producing an elastic laminate which behaves anisotropically upon stretching comprising the steps of:
a. contacting a non-woven fabric web which is elastically extendable in one direction only with a vulcanizable rubber sheet to form a composite laminate;
b. feeding the composite laminate between nip rollers; and
c. vulcanizing the rubber of the composite
laminate to produce an elastic laminate which is elastically stretchable by at least 100% in one direction only.

2. The method according to claim 1, wherein the non-woven fabric web is elastically extendable in the cross-direction only and hence the elastic laminate is elastically stretchable in the cross-direction only.

3. The method according to claim 1 or claim 2, wherein the non-woven fabric web is selected from the group consisting of polyethylene, polypropylene or co polymers of the two.

4. The method according to any preceding claim, wherein the nip rollers are patterned.

5. The method according to any preceding claim wherein the vulcanized rubber sheet includes a crosslinking agent.

6. The method according to any preceding claim, wherein the rubber is vulcanized by subjecting it to conditions under which crosslinking takes place.

7. The method according to claim 6, wherein the composite laminate is heated to a temperature in the range from 100 to 170°C.

8. The method according to any of claims 1 to 6, wherein vulcanization is carried out after step (b) by rolling multiple layers of the composite laminate onto a metal drum and then heating the drum in an oven to a temperature in the range from 100 to 170°C.

9. The method according to any of claims 1 to 6, wherein in step (c), vulcanization is achieved by use of electron beam technology.

10. The method according to any preceding claim, wherein the elastic laminate produced is elastically stretchable by at least 200% in one direction only.

11. The method according to any preceding claim, wherein after step (c), the elastic laminate is apertured by use of a laser, needle punching, ultrasonic treatment or slitting.

12. The method according to any preceding claim, wherein the non-woven fabric web has a basis weight in the range from 10 to 150gm⁻².

13. The method according to any preceding claim, wherein the thickness of the vulcanizable rubber sheet is in the range from 0.05 to 0.2mm.

14. The method according to any preceding claim wherein the vulcanizable rubber sheet has upper and lower faces for contact with the non-woven fabric web, and one face is not in contact with the vulcanizable rubber sheet and the method includes the additional step of coating the face which is not in contact with the rubber sheet with a blocking material.

15. The method according to claim 14, wherein the blocking material is talc.

16. The method according to any preceding claim, wherein the vulcanizable rubber sheet has upper and lower surfaces for contact with the non-woven fabric web and in step (a) there are two feeds of non-woven fabric web which are contacted with the upper and lower surface of the vulcanizable rubber sheet respectively.

17. An elastic laminate which has an elastic stretchability of at least 100% in one direction only comprising:
a vulcanized rubber layer having upper and lower surfaces;
a non-woven fabric layer attached to either or both of the upper and lower surfaces of the vulcanized rubber layer wherein the non-woven fabric web is mechanically entangled with the vulcanized rubber layer.

18. The elastic laminate of claim 17 wherein only one of the upper or lower face is attached to the vulcanized rubber layer and the other is coated with a blocking material.

19. The elastic laminate of claim 18, wherein the blocking material is talc.

20. The elastic laminate of any of claims 17 to 19, which is elastically stretchable in the cross-direction only.

21. Use of the elastic laminate of any of claims 17 to 20, in the production of a disposable hygiene product.

22. Use of the elastic laminate of any of claims 17 to 20 in the production of a disposable medical product.
